# EUROPEAN PATENT APPLICATION

(11) **EP 3 960 241 A1**
(43) Date of publication of application: **02.03.2022**
(21) Application number: 20795639.2
(22) Date of filing: 24.04.2020
(51) Int. Cl.: A61P 31/14, A61K 33/26, A61K 31/197, A61K 31/22

(54) **THERAPEUTIC AGENT FOR FLAVIVIRUS INFECTION**

(30) Priority: 26.04.2019 JP 2019086554
(71) Applicant: Neopharma Japan Co., Ltd., Tokyo 102-0071 (JP); Dokkyo Medical University, Shimotsuga-gun, Tochigi 321-0293 (JP)
(72) Inventor: MASUDA Michiaki, Shimotsugagun, Tochigi 321-0293 (JP); ISHIKAWA Tomohiro, Shimotsugagun, Tochigi 321-0293 (JP); KAWATA Satofumi, Tokyo 102-0071 (JP); TOMIOKA Motoyasu, Tokyo 102-0071 (JP); WADA Yasufumi, Tokyo 102-0071 (JP)
(74) Representative: Wilson Gunn
(86) International application number: PCT/JP2020/017837
(87) International publication number: WO 2020/218577

(57) **Abstract**

Provided are an agent for preventing and/or treating a flavivirus infectious disease and a method for preventing and/or treating a flavivirus infectious disease. At least one selected from 5-aminolevulinic acid (5-ALA), a 5-ALA ester, a salt of 5-ALA, or a salt of the 5-ALA ester is used in the agent for preventing and/or treating the flavivirus infectious disease. Examples of the flavivirus to be prevented or treated by 5-ALA or the like can include dengue virus, Zika virus, Japanese encephalitis virus, tick-borne encephalitis virus, West Nile virus, and yellow fever virus.

## Description

### Technical Field

The present invention generally relates to an agent for preventing and/or treating a flavivirus infectious disease, and a method for preventing and/or treating a flavivirus infectious disease.

### Background Art

The genus Flavivirus of the family Flaviviridae are composed of enveloped viruses having positive-strand RNA as genomes, and most of which are transmitted by blood-sucking arthropods. Examples of mosquito-borne flaviviruses include Japanese encephalitis virus (refer to Non-Patent Literature 1), dengue virus (refer to Non-Patent Literature 2), Zika virus (refer to Non-Patent Literature 3), West Nile virus, and yellow fever virus. Examples of tick-borne flaviviruses include tick-borne encephalitis virus. The genus Flavivirus contains many pathogens that cause infectious diseases considered as a global-scale public health problem, such as Japanese encephalitis, tick-borne encephalitis, dengue hemorrhagic fever, and congenital Zika virus infectious disease. Animals other than humans are reservoirs and amplifier hosts of these viruses, and it is difficult to completely block contact with infected mosquitoes or infected ticks. Thus, developing therapeutic agents specific to these viruses is an important issue (refer to Non-Patent Literature 4). Further, global warming in recent times has caused an increase in lifespans and habitat areas of mosquitoes that transmit flavivirus infectious diseases, fueling concerns over the increased occurrence and spreading of the flavivirus infectious diseases.

In recent years, as functions and structures of flavivirus proteins have become clear, many specific inhibitors have been developed (refer to Non-Patent Literature 5). For example, nitazoxanide (refer to Non-Patent Literature 6) is known to suppress replication of Japanese encephalitis virus. Further, niclosamide or the like (refer to Non-Patent Literature 7) is known to suppress replication of Zika virus. Hemin (refer to Non-Patent Literature 8) is known to suppress replication of Zika virus in vitro. Further, ribavirin (refer to Non-Patent Literatures 9 and 10), lucidone (refer to Non-Patent Literature 11), and a specific indole derivative (refer to Non-Patent Literature 12) are known to suppress replication of dengue virus.

5-Aminolevulinic acid (5-ALA) is present in the mitochondria in cells. It is known that, in animals, 5-ALA is biosynthesized in the mitochondria and serves as an essential component for metabolism, for example, by binding to an iron component and providing a raw material of heme and cytochrome, while, in plants, it is biosynthesized in chloroplasts and serves as an essential component for photosynthesis by binding to magnesium and forming chlorophyll. Further, Patent Literature 1 discloses a method for producing 5-ALA phosphate and also discloses that a method for synthesizing 5-ALA hydrochloride is already known. Further, Patent Literature 2 discloses a method for producing 5-ALA using a microorganism.

Patent Literature 3 discloses an agent for preventing and/or treating an influenza virus infectious disease including 5-ALA. Further, Patent Literature 4 discloses an agent for preventing and/or treating a virus infectious disease including 5-ALA. Patent Literature 4 discloses, as a virus to be treated, Hepatitis B virus, Hepatitis C virus, Ebola virus, human immunodeficiency virus, herpes simplex virus, varicella-zoster virus, and variola virus. Patent Literature 5 discloses a method for treating viral infection of a subject, the method including administering 5-ALA to the subject, accumulating protoporphyrin in cells infected by viruses, and destroying the cells by applying red light to the cells. Patent Literature 6 discloses a use of 5-aminolevulinic acid (5-ALA) hexyl ester or a pharmaceutically acceptable salt thereof in the production of a composition used in a photodynamic therapy (PDT) applied to an animal for treating viral infection in the vaginal cavity, the uterine cervix, or the inner lining of the uterus. However, in Patent Literatures 3 to 6, no studies have been conducted regarding effects of 5-ALA on viruses belonging to the genus Flavivirus of the family Flaviviridae.

Non-Patent Literatures 13, 14, and 15 demonstrate that combined use of 5-ALA and sodium ferrous citrate (SFC) increases the amount of heme oxygenase-1 (HO-1) in cells. Further, Non-Patent Literature 14 demonstrates that 5-ALA alone induces expression of HO-1. Non-Patent Literature 16 demonstrates that HO-1 has antiviral activity against various viruses including dengue virus. Non-Patent Literature 8 demonstrates that hemin exhibiting HO-1 induction activity reduces replication of Zika virus in vitro. Non-Patent Literature 11 demonstrates that lucidone having antiviral activity against dengue virus increases HO-1 in cells.

### Prior Art Literatures

### Patent Literature

Patent Literature 1: JP 2006-182753 A
Patent Literature 2: JP 2005-333907 A
Patent Literature 3: WO 2014/013664 A
Patent Literature 4: WO 2016/163082 A
Patent Literature 5: JP 2000-510123 A
Patent Literature 6: JP 2014-94963 A

### Non-Patent Literature

Non-Patent Literature 1: Sang-Im Yun and Young-Min Lee, Japanese encephalitis The virus and vaccines, Human Vaccines & Immunotherapeutics, 10:2, 263.279; February 2014, p.263-279
Non-Patent Literature 2: Yi-Lin Cheng, et al., Activation of Nrf2 by the dengue virus causes an increase in CLEC5A, which enhances TNF-α production by mononuclear phagocytes, Scientific Reports, 26 August 2016, 6:32000; DOI:10.1038/srep32000
(2016) Non-Patent Literature 3: Oumar Faye, et al., Molecular Evolution of Zika Virus during Its Emergence in the 20th Century, PLOS Neglected Tropical Diseases, January 2014, Volume 8, Issue 1, e2636
Non-Patent Literature 4: Tomohiro ISHIKAWA, Eiji KONISHI, Virus, VOLUME 61, NUMBER 2, 2011, p. 221-238
Non-Patent Literature 5: Veaceslav Boldescu, et al., Broad-spectrum agents for flaviviral infections:dengue, Zika and beyond, NATURE REVIEWS, 5 May 2017, VOLUME 16, AUGUST 2017, p.565-586
Non-Patent Literature 6: Zixue Shi, et al., Nitazoxanide inhibits the replication of Japanese encephalitis virus in cultured cells and in a mouse model, Virology Journal, 2014 Jan 23, 2014; 11:10
Non-Patent Literature 7: Miao Xu, et al., Identification of small-molecule inhibitors of Zika virus infection and induced neural cell death via a drug repurposing screen, NATURE MEDICINE, 29 August 2016, VOLUME 22, NUMBER 10, OCTOBER 2016, p.1101-1107
Non-Patent Literature 8: Hanxia Huang, et al., Nrf2-dependent induction of innate host defense via heme oxygenase-1 inhibits Zika virus replication, Virology. 2017 March, 503, 1-5
Non-Patent Literature 9: W. MARKLAND, et al., Broad-Spectrum Antiviral Activity of the IMP Dehydrogenase Inhibitor VX-497: a Comparison with Ribavirin and Demonstration of Antiviral Additivity with Alpha Interferon, ANTIMICROBIAL AGENTS AND CHEMOTHERAPY, Apr. 2000, Vol. 44, No. 4, p. 859-866
Non-Patent Literature 10: Ratree Takhampunya, et al., Inhibition of dengue virus replication by mycophenolic acid and ribavirin, Journal of General Virology (2006), 87, p.1947-1952
Non-Patent Literature 11: Wei-Chun Chen, et al., Lucidone suppresses dengue viral replication through the induction of heme oxygenase-1, VIRULENCE, 2018, VOL. 9, NO. 1, p.588-603
Non-Patent Literature 12: Dorothee Bardiot, et al., Discovery of Indole Derivatives as Novel and Potent Dengue Virus Inhibitors, Journal of Medicinal Chemistry, August 27, 2018, 2018, 61 (18), DOI:10.1021/acs.jmedchem.8b00913, p. 8390-8401
Non-Patent Literature 13: Mingyi Zhao, et. al., 5-Aminolevulinic acid combined with sodium ferrous citrate ameliorates H2O2-induced cardiomyocyte hypertrophy via activation of the MAPK/Nrf2/HO-1 pathway, American Journal of Physiology Cell Physiology (2015), 308:C665-C672
Non-Patent Literature 14: Kei Saito, et. al., Dynamics of absorption, metabolism, and excretion of 5-aminolevulinic acid in human intestinal Caco-2 cells, Biochemistry and Biophysics Reports, 13 July 2017, 11:105-111
Non-Patent Literature 15: Hidenori Ito, et. al., Oral administration of 5-aminolevulinic acid induces heme oxygenase-1 expression in peripheral blood mononuclear cells of healthy human subjects in combination with ferrous iron, European Journal of Pharmacology, 10 May 2018, 833:25-33
Non-Patent Literature 16: Espinoza JA, Gonzalez PA, Kalergis AM. Modulation of Antiviral Immunity by Heme Oxygenase-1. Am J Pathol. 2017 Mar;187(3):487-493

### Summary of Invention

### Technical Problem

As combating a flavivirus infectious disease becomes more critical, there are demands for a further agent for preventing and/or treating the flavivirus infectious disease. In particular, there are demands for a further agent for preventing and/or treating the flavivirus infectious disease, which has a higher anti-flavivirus activity than the existing compound such as lucidone. Further, there are demands for an agent for preventing and/or treating the flavivirus infectious disease, which has a larger difference in concentrations for exhibiting cell toxicity and drug efficacy (i.e., a therapeutic window) than the existing compound such as lucidone. The present inventors have conducted intensive studies on a further agent for preventing and/or treating the flavivirus infectious disease and found that 5-ALA or an ester thereof, or a salt thereof is highly useful, thereby completing the present invention based on the finding.

### Solution to Problem

That is, some embodiments of the present invention are described as follows.
[1] An agent for preventing and/or treating a flavivirus infectious disease, comprising at least one selected from 5-aminolevulinic acid (5-ALA) or an ester thereof, or a salt thereof.
[2] The agent for preventing and/or treating the flavivirus infectious disease according to [1], in which a flavivirus is a dengue virus, a Zika virus, a Japanese encephalitis virus, a tick-borne encephalitis virus, a West Nile virus, and a yellow fever virus.
[3] The agent for preventing and/or treating the flavivirus infectious disease according to [1], further comprising an iron compound.
[4] The agent for preventing and/or treating the flavivirus infectious disease according to [1], wherein light irradiation is not required.
[5] A method for preventing and/or treating a flavivirus infectious disease, comprising administering an agent for preventing and/or treating the flavivirus infectious disease comprising at least one selected from 5-aminolevulinic acid (5-ALA) or an ester thereof, or a salt thereof to a subject.
[6] The method for preventing and/or treating the flavivirus infectious disease according to [5], in which a flavivirus is a dengue virus, a Zika virus, a Japanese encephalitis virus, a tick-borne encephalitis virus, a West Nile virus, and a yellow fever virus.
[7] The method for preventing and/or treating the flavivirus infectious disease according to [5], in which the agent for preventing and/or treating the flavivirus infectious disease further comprises an iron compound.
[8] The method for preventing and/or treating the flavivirus infectious disease according to [5], wherein light irradiation is not required.

### Advantageous Effects of Invention

The agent for preventing and/or treating a flavivirus infectious disease according to one embodiment of the present invention, which contains at least one selected from 5-aminolevulinic acid (5-ALA) or the ester thereof, or the salt thereof, can suppress replication of the flavivirus, making it possible to obtain an effect of being able to prevent and/or treat the flavivirus infectious disease. The agent for preventing and/or treating the flavivirus infectious disease according to one embodiment of the present invention, which contains at least one selected from 5-aminolevulinic acid (5-ALA) or the ester thereof, or the salt thereof, can exhibit more enhanced anti-flavivirus activity than lucidone, which exhibits antiviral activity presumably through HO-1. Further, the agent for preventing and/or treating the flavivirus infectious disease according to one embodiment of the present invention, which contains at least one selected from 5-aminolevulinic acid (5-ALA) or the ester thereof, or the salt thereof, exhibits an advantageous effect of having a larger difference in concentrations for exhibiting cell toxicity and drug efficacy (i.e., a therapeutic window) as compared with lucidone.

### Brief Description of Drawings

Fig. 1 is a graph showing an inhibitory effect of 5-ALA on a dengue virus replication when cells are treated with 5-ALA before infection.
Fig. 2 is a graph showing the inhibitory effect of 5-ALA on the dengue virus replication when the cells are treated with 5-ALA concurrently with infection.
Fig. 3 is a graph showing the inhibitory effect of 5-ALA on the dengue virus replication when the cells are treated with 5-ALA for 1 day after infection.
Fig. 4 is a graph showing a concentration-dependent inhibitory effect of 5-ALA on the dengue virus replication when the cells are treated with 5-ALA for 1 day after infection.
Fig. 5 is a graph showing a concentration-dependent inhibitory effect of 5-ALA on the Japanese encephalitis virus replication when the cells are treated with 5-ALA for 1 day after infection.
Fig. 6 is a graph showing the inhibitory effect of 5-ALA on the dengue virus replication when the cells are treated with 5-ALA for 7 days after infection.
Fig. 7 is a graph showing an inhibitory effect of 5-ALA on the Japanese encephalitis virus replication when cells are treated with 5-ALA for 7 days after infection.
Fig. 8 is a graph showing the inhibitory effect of 5-ALA on the dengue virus replication when the cells are treated with 5-ALA for 4 days after infection.
Fig. 9 is a graph showing the inhibitory effect of 5-ALA on the dengue virus replication when the cells are treated with 5-ALA for 2 days after infection.
Fig. 10 is a graph showing an inhibitory effect of 5-ALA on the Japanese encephalitis virus replication when cells are treated with 5-ALA for 4 days after infection.
Fig. 11 is a graph showing an inhibitory effect of 5-ALA on the Japanese encephalitis virus replication when cells are treated with 5-ALA for 2 days after infection.
Fig. 12 is a graph showing cell toxicity of 5-ALA when non-infected cells are treated with a 5-ALA solution for 2 days.
Fig. 13 is a graph showing cell toxicity of 5-ALA when non-infected cells are treated with a 5-ALA solution for 4 days.
Fig. 14 is a graph showing cell toxicity of 5-ALA when non-infected cells are treated with a 5-ALA solution for 7 days.
Fig. 15 is a graph showing cell toxicity of lucidone when non-infected cells are treated with a lucidone solution for 7 days.
Fig. 16 is a graph showing an inhibitory effect of 5-ALA or lucidone on dengue virus replication when cells are treated with 5-ALA or 40 µM lucidone for 7 days after infection.
Fig. 17 is a graph showing an inhibitory effect of 5-ALA or lucidone on the Japanese encephalitis virus replication when cells are treated with 5-ALA or 40 µM lucidone for 7 days after infection.
Fig. 18 is a diagram showing optical microscope photographs of cells treated with 0.2 mM 5-ALA or 40 µM lucidone at the time when the treatment are performed for 2 days in the tests shown in Fig. 16 and Fig. 17.
Fig. 19 is a graph showing the inhibitory effect of 5-ALA or lucidone on the dengue virus replication when the cells are treated with 5-ALA or low dose lucidone for 7 days after infection.
Fig. 20 is a graph showing an inhibitory effect of 5-ALA or lucidone on the Japanese encephalitis virus replication when cells are treated with 5-ALA or low dose lucidone for 7 days after infection.
Fig. 21 is a schematic view illustrating timing of a treatment with the agent, a viral infection treatment, and the like in each example.
Fig. 22 is a graph showing the inhibitory effect of 5-ALA on the dengue virus type-1 replication when the cells are treated with 5-ALA for 7 days after infection.
Fig. 23 is a graph showing the inhibitory effect of 5-ALA on the dengue virus type-3 replication when the cells are treated with 5-ALA for 7 days after infection.
Fig. 24 is a graph showing the inhibitory effect of 5-ALA on the dengue virus type-4 replication when the cells are treated with 5-ALA for 7 days after infection.
Fig. 25 is a graph showing the inhibitory effect of 5-ALA on the Zika virus replication when the cells are treated with 5-ALA for 7 days after infection.

### Description of Embodiments

One embodiment of the present invention is an agent for preventing and/or treating a flavivirus infectious disease comprising at least one selected from 5-aminolevulinic acid (5-ALA) or an ester thereof, or a salt thereof. Further, one embodiment of the present invention is a use of at least one selected from 5-aminolevulinic acid (5-ALA) or an ester thereof, or a salt thereof in an agent for preventing and/or treating a flavivirus infectious disease. Further, another embodiment of the present invention is a composition for preventing and/or treating a flavivirus infectious disease, comprising at least one selected from 5-aminolevulinic acid (5-ALA) or an ester thereof, or a salt thereof.

In the present invention, 5-aminolevulinic acid (5-ALA) is a compound also referred to as δ-aminolevulinic acid. In the present invention, "5-ALA or an ester thereof' refers to "5-ALA or a 5-ALA ester" and is represented by the following Formula (I). In the present invention, "a salt thereof' in the description of "5-ALA or an ester thereof, or a salt thereof' refers to a salt of 5-ALA or a salt of a 5-ALA ester. Examples of such a salt include an acid addition salt such as a hydrochloride salt, a hydrobromide salt, a hydroiodide salt, a phosphate salt, a methylphosphoric acid, an ethylphosphoric acid, a phosphite salt, a hypophosphite salt, a nitrate salt, a sulfate salt, an acetate salt, a propionate salt, a toluenesulfonate salt, a succinate salt, an oxalate salt, a lactate salt, a tartrate salt, a glycolate salt, a methanesulfonate salt, a butyrate salt, a valerate salt, a citrate salt, a fumarate salt, a maleate salt, or a malate salt, and a metal salt such as a sodium salt, a potassium salt, or a calcium salt, an ammonium salt, and an alkyl ammonium salt, without being limited thereto.

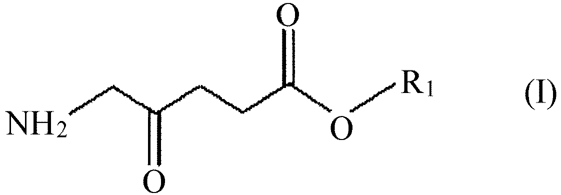

In the above Formula (I), R₁ represents a hydrogen atom, a linear or branched alkyl group, a cycloalkyl group, an aryl group, or an aralkyl group. In a case where R₁ is hydrogen, the above Formula (I) represents 5-ALA. In a case where R₁ is a linear or branched alkyl group, a cycloalkyl group, an aryl group, or an aralkyl group, the above Formula (I) represents a 5-ALA ester.

The linear or branched alkyl group represented by R₁ is preferably an alkyl group having 1 to 18 carbon atoms, and examples thereof include a methyl group, an ethyl group, a n-propyl group, an isopropyl group, a n-butyl group, an isobutyl group, a tert-butyl group, a n-pentyl group, an isopentyl group, a neopentyl group, a tert-pentyl group, a 2-methylbutyl group, a n-hexyl group, an isohexyl group, a 3-methylpentyl group, an ethylbutyl group, a n-heptyl group, a 2-methylhexyl group, a n-octyl group, an isooctyl group, a tert-octyl group, a 2-ethylhexyl group, a 3-methylheptyl group, a n-nonyl group, an isononyl group, a 1-methyloctyl group, an ethylheptyl group, a n-decyl group, a 1-methylnonyl group, a n-undecyl group, a 1,1-dimethylnonyl group, a n-dodecyl group, a n-tridecyl group, a n-tetradecyl group, a n-pentadecyl group, a n-hexadecyl group, a n-heptadecyl group, and an n-octadecyl group. Examples of the cycloalkyl group not only include a cyclopropyl group, a cyclobutyl group, a cyclopentyl group, a cyclohexyl group, a cycloheptyl group, and a cyclooctyl group, but also include a cycloalkyl group having an alkyl substituent, for example, a cycloalkyl group having an alkyl substituent having 1 to 6 carbon atoms, for example, a 3-methylcyclohexyl group, a 4-methylcyclohexyl group, a 4-ethylcyclohexyl group, and a 2-methylcyclooctyl group. As the linear or branched alkyl group, an alkyl group having 1 to 16 carbon atoms is preferable, and a methyl group, an ethyl group, a n-butyl group, a n-hexadecyl group, or a 2-ethylhexyl group is particularly preferable.

The aryl group represented by R₁ may include a phenyl group, a naphthyl group, and the like. The aryl group may be substituted by 1 to 3 substituent(s) such as, for example, an alkyl group having 1 to 6 carbon atoms such as a methyl group, an ethyl group, a n-propyl group, an isopropyl group, a n-butyl group, an isobutyl group, a tert-butyl group, a n-pentyl group, a n-hexyl group, a cyclopropyl group, a cyclobutyl group, or a cyclohexyl group, an alkoxy group having 1 to 6 carbon atoms such as a methoxy group, an ethoxy group, a n-propoxy group, a n-butoxy group, an isobutoxy group, or a tert-butoxy group, a hydroxyl group, an amino group, a nitro group, a cyano group, a halogen atom such as fluorine, chlorine, bromine, or iodine, and a carboxyl group.

The aralkyl group represented by R₁ is preferably constituted by an alkyl group having 1 to 6 carbon atoms and an aryl group having 6 to 20 carbon atoms. Examples of the alkyl group having 1 to 6 carbon atoms include a methyl group, an ethyl group, a n-propyl group, an isopropyl group, a n-butyl group, an isobutyl group, a tert-butyl group, a n-pentyl group, an n-hexyl group, a cyclopropyl group, a cyclobutyl group, and a cyclohexyl group. Examples of the aryl group having 6 to 20 carbon atoms include a phenyl group and a naphthyl group. Among the aralkyl groups, a benzyl group or a phenethyl group is preferable, and a benzyl group is particularly preferable. The aryl group of the aralkyl groups may be substituted by 1 to 3 substituent(s) such as, for example, the above-mentioned alkyl group having 1 to 6 carbon atoms, an alkoxy group having 1 to 6 carbon atoms such as a methoxy group, an ethoxy group, a n-propoxy group, a n-butoxy group, an isobutoxy group, or a tert-butoxy group, a hydroxyl group, an amino group, a nitro group, a cyano group, a halogen atom such as fluorine, chlorine, bromine, or iodine, and a carboxyl group.

In the agent for preventing and/or treating the flavivirus infectious disease of the embodiment of the present invention, at least one selected from 5-ALA or the ester thereof, or the salt thereof may be used as an active component, and the active components may be used singly or in combination of two or more thereof. For example, the active component used in the present embodiment may be any of 5-ALA, the 5-ALA ester, the 5-ALA salt, or the salt of the 5-ALA ester. Further, for example, the active component may be a combination of 5-ALA and the salt of the 5-ALA ester, or the like. At least the one selected from 5-ALA or the ester thereof, or the salt thereof used in the present embodiment may be a purified product, a crudely purified product, or a product obtained as a mixture by synthesis. In the present embodiment, the 5-ALA salt is preferably used as the active component, and 5-ALA hydrochloride and/or 5-ALA phosphate is more preferably used as the active component. At least the one selected from 5-ALA or the ester thereof, or the salt thereof used in the present invention can be produced by a publicly known method.

In the present invention, "flavivirus" refers to a virus belonging to the genus Flavivirus of the family Flaviviridae. Examples of the flavivirus include dengue virus, Zika virus, Japanese encephalitis virus, West Nile virus, yellow fever virus, Murray Valley encephalitis virus, Saint Louis encephalitis virus, Omsk hemorrhagic fever virus, and tick-borne encephalitis virus. In one embodiment of the present invention, the flavivirus to be prevented and/or treated is a virus belonging to the genus Flavivirus of the family Flaviviridae. The flavivirus to be prevented and/or treated is preferably dengue virus, Zika virus, Japanese encephalitis virus, tick-borne encephalitis virus, West Nile virus, or yellow fever virus, more preferably dengue virus, Zika virus, or Japanese encephalitis virus.

In the present invention, "flavivirus infectious disease" refers to a disease caused by the flavivirus infecting humans, animals other than human, and the like. Examples of the flavivirus infectious disease in humans include dengue fever, dengue hemorrhagic fever, and dengue shock syndrome caused by infection with dengue virus, Zika virus disease and congenital Zika virus infectious disease caused by infection with Zika virus, Japanese encephalitis caused by infection with Japanese encephalitis virus, West Nile fever and West Nile encephalitis caused by infection with West Nile virus, yellow fever caused by infection with yellow fever virus, Murray Valley encephalitis caused by infection with Murray Valley encephalitis virus, Saint Louis encephalitis caused by infection with Saint Louis encephalitis virus, Omsk hemorrhagic fever caused by infection with Omsk hemorrhagic fever virus, and tick-borne encephalitis caused by infection with tick-borne encephalitis virus. Further, examples of the flavivirus infectious disease in animals other than humans include encephalitis caused by Japanese encephalitis virus infecting a horse, miscarriage caused by Japanese encephalitis virus infecting a pregnant pig, encephalitis caused by West Nile virus infecting a horse, and miscarriage caused by West Nile virus infecting a pregnant sheep, without being limited thereto.

In the present invention, "preventing the flavivirus infectious disease" includes, for example, inhibiting the onset of the flavivirus infectious disease that is, completely inhibiting the onset thereof or reducing a rate of the onset thereof, without being limited thereto. Further, "treating the flavivirus infectious disease" includes, for example, preventing aggravated conditions of the flavivirus infectious disease, ameliorating conditions thereof, and completely curing the flavivirus infectious disease, without being limited thereto. In the present invention, "preventing and/or treating" means any of the following (1) to (3): (1) preventing, (2) treating, and (3) both preventing and treating.

In one embodiment of the present invention, the agent for preventing and/or treating the flavivirus infectious disease may adopt any administration route, dosage form, and composition as long as the infectious disease of a subject to be administered can be prevented and/or treated. For example, in one embodiment of the present invention, the agent for preventing and/or treating the flavivirus infectious disease may be administered by various routes including oral administration, intravenous administration, intranasal administration, transdermal administration, inhalation administration, administration in a suppository form, or the like, without being limited thereto. The administration route of the agent for preventing and/or treating the flavivirus infectious disease is preferably oral administration. In one embodiment of the present invention, the agent for preventing and/or treating the flavivirus infectious disease can adopt a dosage form suitable for the administration route. Examples of the dosage form can include a form of a tablet, a powder, a capsule, an elixir, a suspension, an emulsion, a solution, syrup, an ointment, a suppository, and a patch. Further, in one embodiment of the present invention, the agent for preventing and/or treating the flavivirus infectious disease may be in a form of food. Further, in one embodiment of the present invention, the agent for preventing and/or treating the flavivirus infectious disease may be in a form of feed provided to animals other than humans.

In one embodiment of the present invention, the agent for preventing and/or treating the flavivirus infectious disease comprises an iron compound. The iron compound is not particularly limited, and examples of the iron compound can include an organic salt of iron, an inorganic salt of iron, or a complex with a polymeric compound such as a protein. Examples of the organic salt of iron include a citric acid salt such as ferrous citrate, iron sodium citrate, sodium ferrous citrate, or iron ammonium citrate, a hydroxycarboxylic acid salt such as iron malate, iron sodium succinate citrate, iron lactate, iron tartrate, or iron glycolate, ferrous succinate, iron acetate, iron oxalate, iron dextran, iron gluconate, iron sodium ethylenediaminetetraacetate, iron potassium ethylenediaminetetraacetate, iron ammonium ethylenediaminetetraacetate, iron sodium diethylenetriaminepentaacetate, iron potassium diethylenetriaminepentaacetate, iron ammonium diethylenetriaminepentaacetate, and iron glycerophosphate. Examples of the inorganic salt of iron include iron oxide, iron chloride, iron nitrate, iron sulfate, ammonium iron sulfate, ferrous pyrophosphate, and ferric pyrophosphate. Further, the iron compound may be an iron-binding protein such as lactoferrin iron or transferrin iron, heme iron, or the like. The iron compounds may be used singly or in combination of two or more thereof. The iron compound is preferably sodium ferrous citrate.

In one embodiment of the present invention, as the amount of the iron compound contained in the agent for preventing and/or treating the flavivirus infectious disease, a ratio of the total molar amount of 5-aminolevulinic acid (5-ALA) or the ester thereof, or a salt thereof:the molar amount of iron atoms in the iron compound is from 1:0.05 to 1:5, preferably from 1:0.1 to 1:1.

In one embodiment of the present invention, the agent for preventing and/or treating the flavivirus infectious disease can include as needed various pharmaceutically or food-hygienically acceptable carriers and additives or various carriers and additives acceptable as feed for animals other than humans, etc. Examples of these various carriers and additives can include an excipient, a lubricant, a stabilizer, a dispersant, a binder, a diluent, a flavoring agent, a sweetener, a seasoning, and a colorant. In one embodiment of the present invention, the agent for preventing and/or treating the flavivirus infectious disease can be a composition for preventing and/or treating the flavivirus infectious disease including these necessary components.

Another embodiment of the present invention is a method for preventing and/or treating the flavivirus infectious disease which comprises administering the agent for preventing and/or treating the flavivirus infectious disease comprising at least one selected from 5-aminolevulinic acid (5-ALA) or the ester thereof, or the salt thereof to a subject. Further, another embodiment of the present invention is a use of at least one selected from 5-aminolevulinic acid (5-ALA) or the ester thereof, or the salt thereof in a subject to be prevented from and/or treated for the flavivirus infectious disease. The subject to be prevented from and/or treated for the flavivirus infectious disease in the method for preventing and/or treating the flavivirus infectious disease of the embodiment of the present invention is not particularly limited, and humans, mammals other than humans, and birds can be mentioned.

A dose of 5-aminolevulinic acid (5-ALA) or the ester thereof, or the salt thereof in the method for preventing and/or treating the flavivirus infectious disease of the present invention can be appropriately determined according to the kind of viruses, the seriousness of symptoms, and the like. A frequency and period of administration is not particularly limited.

In one embodiment of the present invention, the agent for preventing and/or treating the flavivirus infectious disease is an agent for preventing and/or treating the flavivirus infectious disease which comprises at least one selected from 5-aminolevulinic acid (5-ALA) or the ester thereof, or the salt thereof and does not require light irradiation. In another embodiment, the agent for preventing and/or treating the flavivirus infectious disease is an agent for preventing and/or treating the flavivirus infectious disease which comprises at least one selected from 5-aminolevulinic acid (5-ALA) or the ester thereof, or the salt thereof and is used without light irradiation. Further, in one embodiment of the present invention, the method for preventing and/or treating the flavivirus infectious disease is a method for preventing and/or treating the flavivirus infectious disease which comprises administering the agent for preventing and/or treating the flavivirus infectious disease including at least one selected from 5-aminolevulinic acid (5-ALA) or the ester thereof, or the salt thereof to a subject and does not require light irradiation. In another embodiment, the method for preventing and/or treating the flavivirus infectious disease is a method for preventing and/or treating the flavivirus infectious disease which comprises administering the agent for preventing and/or treating the flavivirus infectious disease comprising at least one selected from 5-aminolevulinic acid (5-ALA) or the ester thereof, or the salt thereof to a subject, in which light irradiation is not performed. The "light irradiation" in these embodiments may include, for example, light irradiation used in a photodynamic therapy (PDT) that utilizes a photosensitivity of protoporphyrin (protoporphyrin IX) accumulated in cells.

Hereinafter, the present invention will be described in detail by way of examples, but the present invention is not limited to the range of the examples.

### Examples

Cells were treated with agents such as 5-ALA before, concurrently with, or after infection with flaviviruses and inhibitory effects of the agents on replication of dengue virus (DENV) or Japanese encephalitis virus (JEV) were confirmed according to the following procedures.
(1) One day before infection (Day 0), Vero cells were seeded in a 24-well plate and incubated at 37°C and 5% CO₂. A culture solution was MEM + 10% fetal bovine serum + 10 mM non-essential amino acid + 1000 U/ml penicillin + 100 µg/ml streptomycin. In an experiment in which the treatment with the agent was performed before infection, the agent to be examined was added to the culture solution at this point. In all experiments, 5-aminolevulinic acid hydrochloride was used as 5-ALA, sodium ferrous citrate (SFC) was used as an iron-containing compound, 300 µM ribavirin or lucidone of various concentrations was used as a positive control, and water was used as a negative control.
(2) On the day of infection (Day 1), the culture solution was removed from the plate thus prepared, and a dengue virus type-2 New Guinea C strain (DENV2 NGC) and a Japanese encephalitis virus Nakayama strain (JEV Nakayama) were each inoculated into the cells at MOI (multiplicity of infection) = 2 and MOI = 5 (an inoculation liquid volume of 200 µl), respectively. After the viruses were adsorbed to the cells for 1 hour, the cells were rinsed with phosphate-buffered saline (PBS) to completely remove the inoculation liquid. A maintenance culture solution (MEM + 1% fetal bovine serum + 10 mM non-essential amino acid + 1000 U/ml penicillin + 100 µg/ml streptomycin + 1 mM HEPES) in a volume of 1 ml was added to the plate, and the cells were cultured at 37°C and 5% CO₂. In an experiment in which the agent was added concurrently with infection, the agent to be examined was added to the maintenance culture solution at this point.
(3) After 24 hours (Day 2), the culture solution was recovered from the plate and a fresh maintenance culture solution in a volume of 1 ml was added to the plate, and the cells were cultured at 37°C and 5% CO₂. In an experiment in which the agent was added after infection, the agent to be examined was added at predetermined time points after infection. The recovered culture solution was subjected to centrifugation at 3000 rpm for 5 minutes and the resulting supernatant was frozen and stored.
(4) Thereafter, recovering and supplying of the culture solution was repeated every 24 hours. It is of note that the infection experiments were performed using 2 wells for all conditions.

The infection titer of the virus was measured according to the following procedures.
(1) One day before the experiment, Vero cells were inoculated in a 24-well plate and incubated at 37°C and 5% CO₂.
(2) Samples for titer measurement were thawed and ten-fold serial dilutions of the samples were prepared using the maintenance culture solution.
(3) The culture solution was removed from the plate thus prepared, and the serial dilutions prepared in procedure (2) were each inoculated in a volume of 200 µl. After adsorption for 1 hour, the inoculation liquid was removed from the plate, and 1% methyl cellulose (MEM + 1% methyl cellulose + 1% fetal bovine serum) in a volume of 1 ml was added to the plate. The plate was left standing still at 37°C and 5% CO₂ (for 14 days for DENV, 10 days for JEV).
(4) The methyl cellulose solution was removed from the plate, and, after rinsing with PBS, crystal violet (0.05% crystal violet and 20% methanol) in a volume of 1 ml was added to the plate. The plate was left standing still at room temperature for 10 minutes. The crystal violet solution was removed from the plate, and, after rinsing with water and drying, the number of plaques was counted. The viral titer in the culture solution was calculated based on the number of plaques.

### MTT assay

(1) One day before the experiment, Vero cells were inoculated in a 96-well plate and incubated at 37°C and 5% CO₂.
(2) Two-fold serial dilutions of 5-ALA were prepared (-0.8 mM) using the maintenance culture solution (MEM + 1% fetal bovine serum + 10 mM non-essential amino acid + 1000 U/ml penicillin + 100 µg/ml streptomycin + 1 mM HEPES). Water was used as a negative control. Two-fold serial dilutions of lucidone were also prepared (-160 µM) in the same manner.
(3) The culture solution was removed from the plate thus prepared, and the 5-ALA solution or the lucidone solution prepared in (2) was added to the plate in a volume of 100 µl per well. Three wells were used for each condition.
(4) The old culture solution was removed from the plate every 24 hours, and the fresh 5-ALA solution or lucidone solution was added to the plate, followed by culturing.
(5) The cells were cultured with the 5-ALA solution for 2, 4, or 7 days or with the lucidone solution for 7 days, and then rinsed with PBS twice. An MTT solution in a volume of 100 µl was added to the cells, and the cells were cultured at 37°C and 5% CO2 for 3 hours. The MTT solution in use was prepared by dissolving thiazolyl blue tetrazolium bromide in a mixture containing equal volumes of 2 x MEM and sterile water (10 mg/ml) and sterilizing the resulting mixture by filtration (prepared before use).
(6) The culture solution was removed from the plate, and isopropyl alcohol in a volume of 100 µl was added to the plate, and the plate was subject to shaking at room temperature for about 5 minutes.
(7) The absorbance at 570 nm was measured, and the relative absorbance was calculated as a percentage with respect to the absorbance in the well of 0 mM treatment.

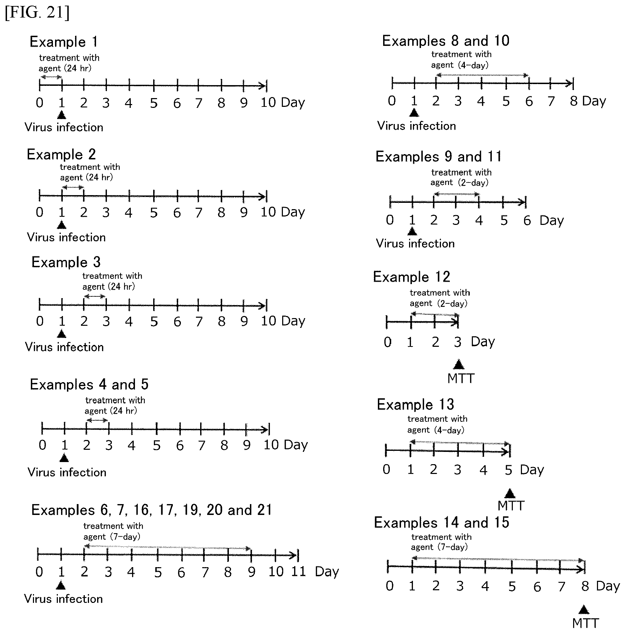

Fig. 21 illustrates timing of a treatment with the agent, a viral infection treatment, and the like in each example as follows.

### Example 1

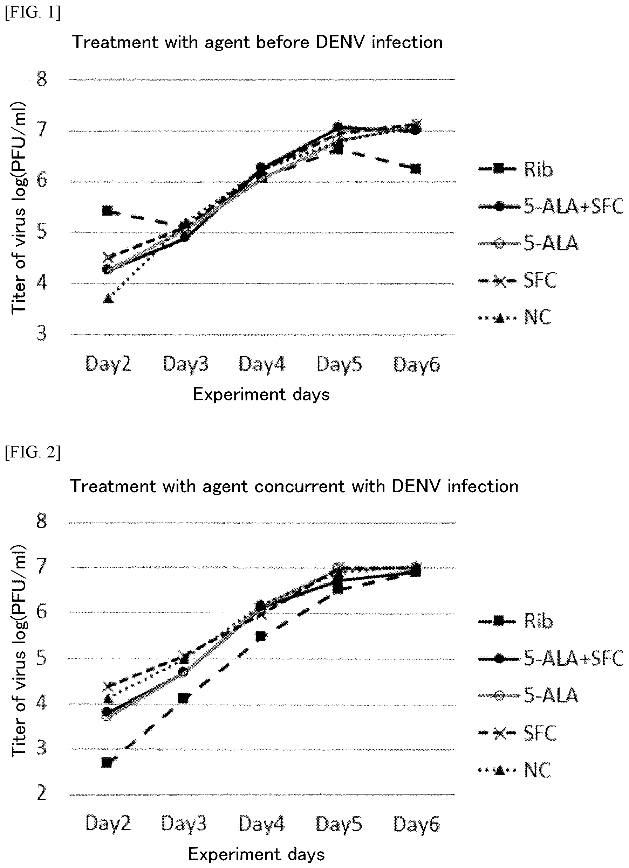

The inhibitory effect on dengue virus replication by treatment with the agent before infection was examined. The agents in use were 5-ALA (1 mM), 5-ALA (1 mM) + SFC (0.25 mM) (i.e., a combined use of 5-ALA and SFC), SFC (0.25 mM), ribavirin (300 µM, Rib in figures), and water (NC in figures). The titer of progeny viruses released from cells treated with the agent was evaluated and plotted on log(PFU/ml) (PFU: plaque-forming unit) as shown in Fig. 1. As shown in Fig. 1, the inhibitory effect on the dengue virus replication was not observed with 5-ALA (1 mM) and 5-ALA (1 mM) + SFC (0.25 mM) administered before infection.

### Example 2

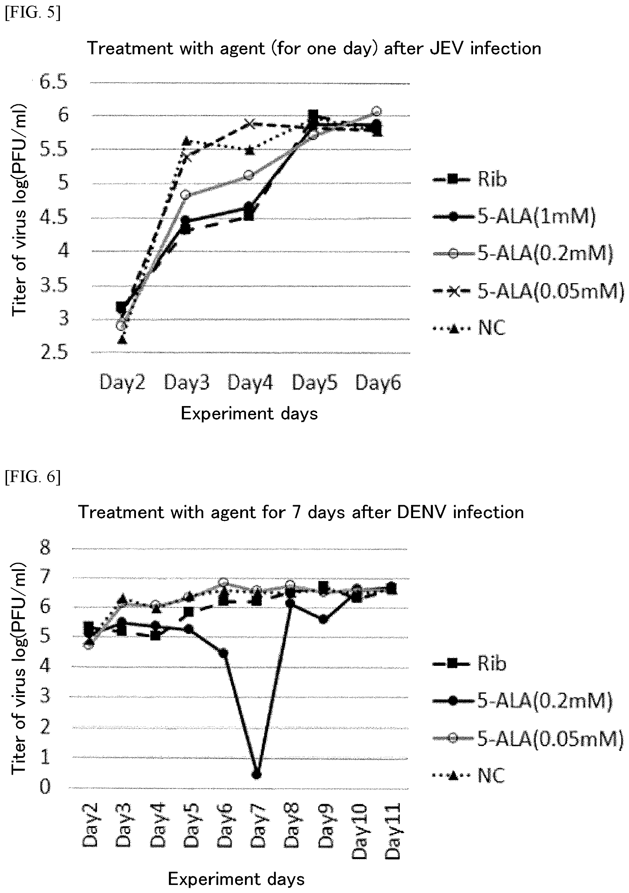

The inhibitory effect on dengue virus replication by treatment with the agent concurrently with infection was examined. The agents in use were 5-ALA (1 mM), 5-ALA (1 mM) + SFC (0.25 mM), SFC (0.25 mM), ribavirin (300 µM, Rib in figures), and water (NC in figures). Treatment with the agent was performed concurrently with infection for 1 day (Day 1). The titer of progeny viruses released from cells treated was evaluated and plotted on log(PFU/ml) (PFU: plaque-forming unit) as shown in Fig. 2. As shown in Fig. 2, 5-ALA (1 mM) and 5-ALA (1 mM) + SFC (0.25 mM) exhibited the inhibitory effect on the dengue virus replication when treatment was performed concurrently with infection. When the agent was removed from the culture solution, this inhibitory effect was lost with the lapse of time.

### Example 3

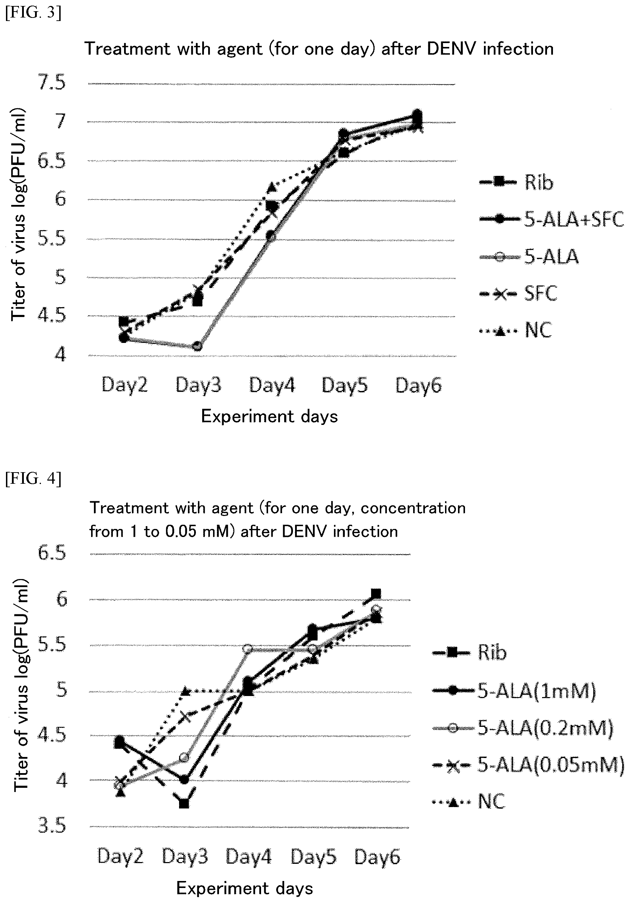

The inhibitory effect on dengue virus replication by treatment with the agent after infection was examined. The agents in use were 5-ALA (1 mM), 5-ALA (1 mM) + SFC (0.25 mM), SFC (0.25 mM), ribavirin (300 µM, Rib in figures), and water (NC in figures). Treatment with the agent was performed from one day after infection for 1 day (Day 2). The titer of progeny viruses released from cells treated was evaluated and plotted on log(PFU/ml) (PFU: plaque-forming unit) as shown in Fig. 3. As shown in Fig. 3, 5-ALA (1 mM) and 5-ALA (1 mM) + SFC (0.25 mM) exhibited the inhibitory effect on the dengue virus replication when treatment was performed after infection. When the agent was removed from the culture solution, this inhibitory effect was lost with the lapse of time.

### Example 4

The dose-dependent inhibitory effect on dengue virus replication by treatment with the agent after infection was examined. The agents in use were 5-ALA (1 mM), 5-ALA (0.2 mM), 5-ALA (0.05 mM), ribavirin (300 µM, Rib in figures), and water (NC in figures). Treatment with the agent was performed from one day after infection for 1 day (Day 2). The titer of progeny viruses released from cells treated was evaluated and plotted on log(PFU/ml) as shown in Fig. 4. As shown in Fig. 4, 5-ALA exhibited the dose-dependent inhibitory effect on the dengue virus replication in a concentration from 0.05 mM to 1 mM in treatment after infection. When the agent was removed from the culture solution, this inhibitory effect was lost with the lapse of time.

### Example 5

The inhibitory effect on the Japanese encephalitis virus replication by treatment with the agent after infection was examined. The agents in use were 5-ALA (1 mM), 5-ALA (0.2 mM), 5-ALA (0.05 mM), ribavirin (300 µM, Rib in figures), and water (NC in figures). Treatment with the agent was performed from one day after infection for 1 day (Day 2). The titer of progeny viruses released from cells treated was evaluated and plotted on log(PFU/ml) as shown in Fig. 5. As shown in Fig. 5, 5-ALA exhibited the dose-dependent inhibitory effect on the Japanese encephalitis virus replication in a concentration from 0.05 mM to 1 mM in treatment after infection. When the agent was removed from the culture solution, this inhibitory effect was lost with the lapse of time. However, the inhibitory effect lasted longer than in the case of the dengue virus in Example 4.

### Example 6

The inhibitory effect on dengue virus replication by treatment with the agent for 7 days after infection was examined. The agents in use were 5-ALA (0.2 mM), 5-ALA (0.05 mM), ribavirin (300 µM, Rib in figures), and water (NC in figures). Treatment with the agent was performed from one day after infection for 7 days (from Day 2 to the end of Day 8). The titer of progeny viruses released from cells treated was evaluated and plotted on log(PFU/ml) as shown in Fig. 6. As shown in Fig. 6, treatment with 5-ALA (0.2 mM) exhibited the inhibitory effect on the dengue virus replication which increased up to Day 7 with the lapse of treatment time. The inhibitory effect on the dengue virus replication by treatment with 5-ALA (0.2 mM) was stronger than the effect of ribavirin (300 µM). On Day 8, the inhibitory effect on the dengue virus replication by treatment with 5-ALA (0.2 mM) decreased. It is thought that the appearance of a 5-ALA resistance virus is one of the causes for the reduction in the inhibitory effect. The reduction in the inhibitory effect caused by continuous treatment with the agent was also observed in treatment with ribavirin.

### Example 7

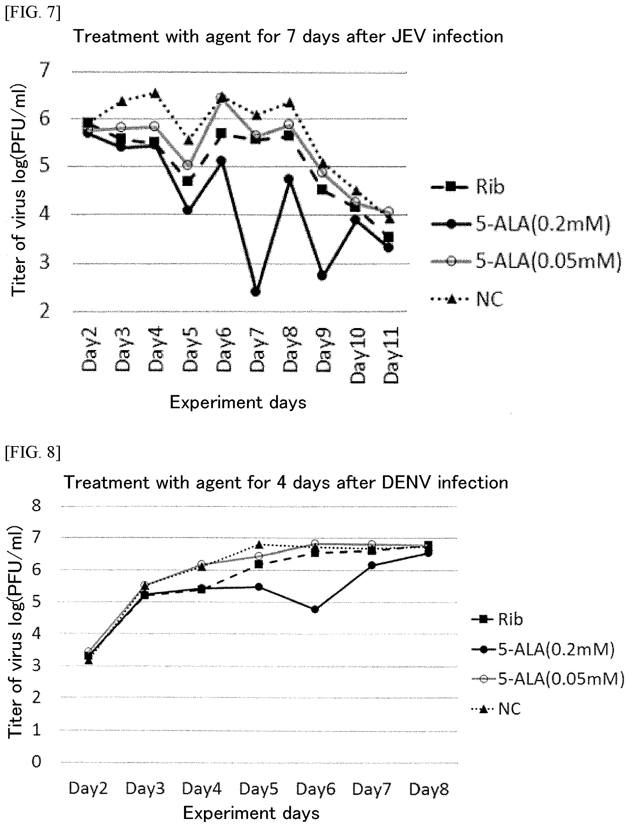

The inhibitory effect on the Japanese encephalitis virus replication by treatment with the agent for 7 days after infection was examined. The agents in use were 5-ALA (0.2 mM), 5-ALA (0.05 mM), ribavirin (300 µM, Rib in figures), and water (NC in figures). Treatment with the agent was performed from one day after infection for 7 days (from Day 2 to the end of Day 8). The titer of progeny viruses released from cells treated was evaluated and plotted on log(PFU/ml) as shown in Fig. 7. As shown in Fig. 7, the inhibitory effect on the Japanese encephalitis virus replication was exhibited by treatment with 5-ALA (0.2 mM and 0.05 mM). The inhibitory effect on the Japanese encephalitis virus replication by treatment with 5-ALA (0.2 mM) was stronger than the effect of ribavirin (300 µM). A reduction in the inhibitory effect on the Japanese encephalitis virus replication was observed in continuous treatment with the agent, and it is thought that the appearance of the Japanese encephalitis virus resistant to the agent is one of the causes for such a reduction. From the result in Fig. 7, it is understood that 5-ALA delays the appearance of the virus resistant to the agent more than ribavirin.

### Example 8

The inhibitory effect on dengue virus replication by treatment with the agent for 4 days after infection was examined. The agents in use were 5-ALA (0.2 mM), 5-ALA (0.05 mM), ribavirin (300 µM, Rib in figures), and water (NC in figures). Treatment with the agent was performed from one day after infection for 4 days (from Day 2 to the end of Day 6). The titer of progeny viruses released from cells treated was evaluated and plotted on log(PFU/ml) as shown in Fig. 8. As shown in Fig. 8, the inhibitory effect on the dengue virus replication was exhibited by treatment with 5-ALA (0.2 mM). When the agent was removed from the culture solution, this inhibitory effect was lost with the lapse of time.

### Example 9

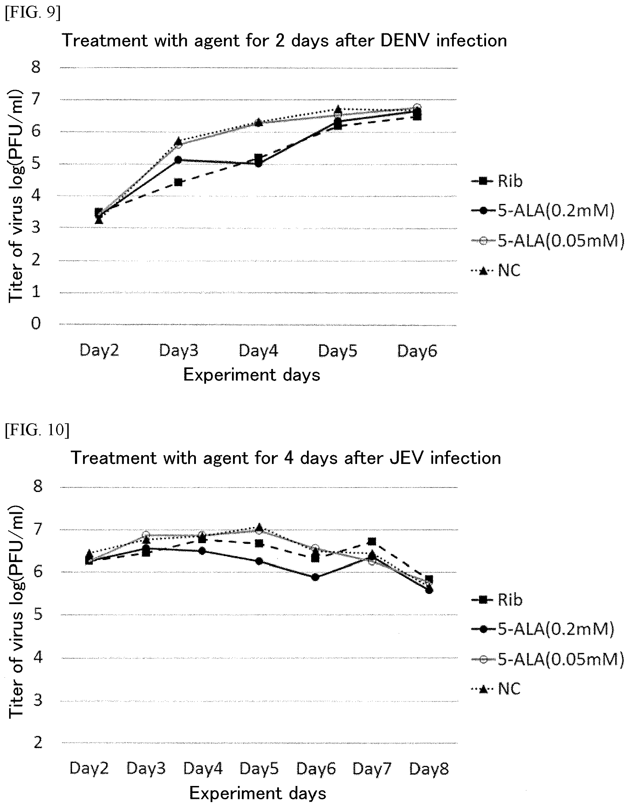

The inhibitory effect on dengue virus replication by treatment with the agent for 2 days after infection was examined. The agents in use were 5-ALA (0.2 mM), 5-ALA (0.05 mM), ribavirin (300 µM, Rib in figures), and water (NC in figures). Treatment with the agent was performed from one day after infection for 2 days (from Day 2 to the end of Day 4). The titer of progeny viruses released from cells treated was evaluated and plotted on log(PFU/ml) as shown in Fig. 9. As shown in Fig. 9, the inhibitory effect on the dengue virus replication was exhibited by treatment with 5-ALA (0.2 mM). When the agent was removed from the culture solution, this inhibitory effect was lost with the lapse of time. From the results in Fig. 6, Fig. 8, and Fig. 9, it was found that treatment with 5-ALA for 4 days and 2 days exhibited less inhibitory effect on the dengue virus replication than treatment with 5-ALA for 7 days.

### Example 10

The inhibitory effect on the Japanese encephalitis virus replication by treatment with the agent for 4 days after infection was examined. The agents in use were 5-ALA (0.2 mM), 5-ALA (0.05 mM), ribavirin (300 µM, Rib in figures), and water (NC in figures). Treatment with the agent was performed from one day after infection for 4 days (from Day 2 to the end of Day 6). The titer of progeny viruses released from cells treated was evaluated and plotted on log (PFU/ml) as shown in Fig. 10. As shown in Fig. 10, the inhibitory effect on the Japanese encephalitis virus replication was exhibited by treatment with 5-ALA (0.2 mM). When the agent was removed from the culture solution, this inhibitory effect was lost with the lapse of time.

### Example 11

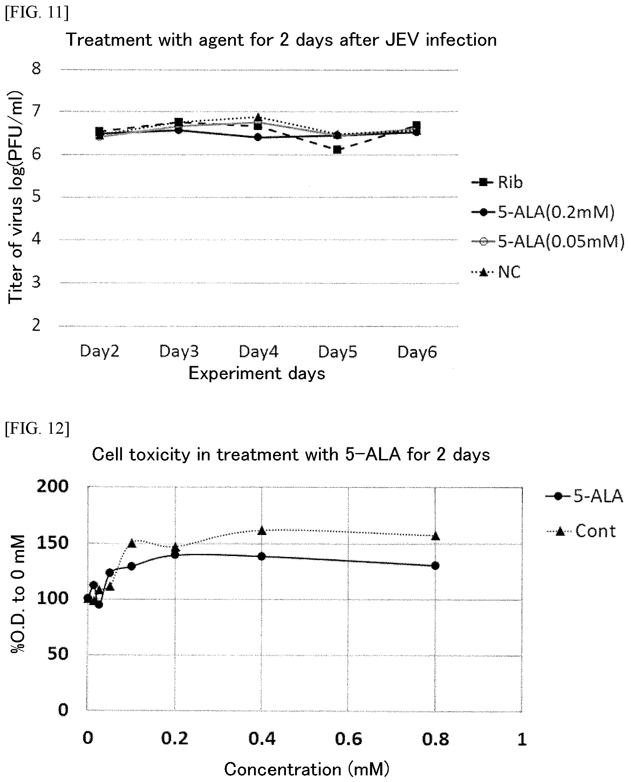

The inhibitory effect on the Japanese encephalitis virus replication by treatment with the agent for 2 days after infection was examined. The agents in use were 5-ALA (0.2 mM), 5-ALA (0.05 mM), ribavirin (300 µM, Rib in figures), and water (NC in figures). Treatment with the agent was performed from one day after infection for 2 days (from Day 2 to the end of Day 4). The titer of progeny viruses released from cells treated was evaluated and plotted on log(PFU/ml) as shown in Fig. 11. As shown in Fig. 11, the inhibitory effect on the Japanese encephalitis virus replication was exhibited by treatment with 5-ALA (0.2 mM). When the agent was removed from the culture solution, this inhibitory effect was lost with the lapse of time. From the results in Fig. 7, Fig. 10, and Fig. 11, it was found that treatment with 5-ALA for 4 days and 2 days exhibited less inhibitory effect on the Japanese encephalitis virus replication than treatment with 5-ALA for 7 days.

### Example 12

Cell toxicity caused to non-infected cells treated with the 5-ALA solution for 2 days was examined according to the above MTT assay. Fig. 12 shows the result of the cell toxicity caused by treatment with the 5-ALA solution for 2 days.

### Example 13

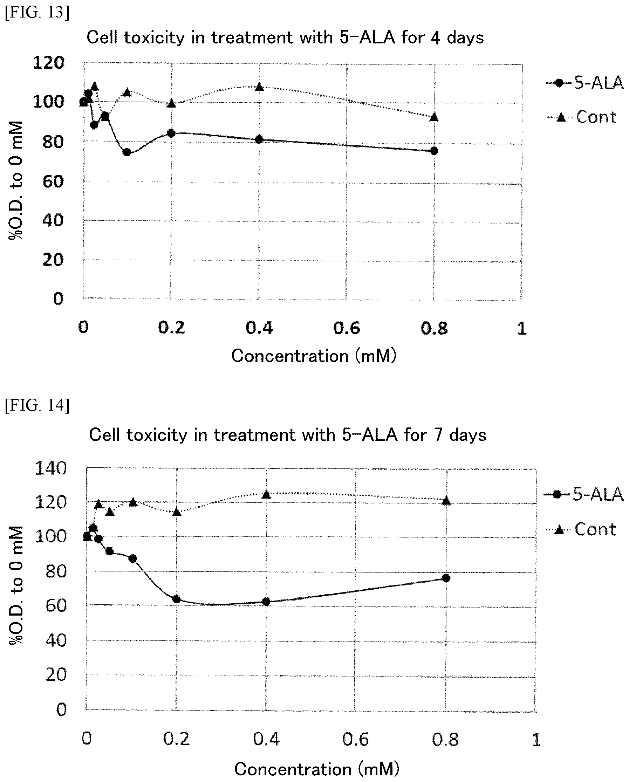

Cell toxicity caused to non-infected cells treated with the 5-ALA solution for 4 days was examined according to the above MTT assay. Fig. 13 shows the result of the cell toxicity caused by treatment with the 5-ALA solution for 4 days.

### Example 14

Cell toxicity caused to non-infected cells treated with the 5-ALA solution for 7 days was examined according to the above MTT assay. Fig. 14 shows the result of the cell toxicity caused by treatment with the 5-ALA solution for 7 days. In Figs. 12, 13, and 14, "Cont" represents a negative control. The cell toxicity of 5-ALA was observed in the non-infected cells in a dose-dependent manner. CC50 (a concentration of an agent at which cell toxicity is caused to 50% of cells) in treatment with 5-ALA for 7 days was higher than 0.8 mM. This concentration, 0.8 mM, was higher than the concentration of 5-ALA, 0.2 mM, at which the inhibitory effect on the virus replication was observed in Examples 6 and 7. This leads to the speculation that 5-ALA can prevent and/or treat the flavivirus infectious disease while minimizing side effects caused by the cell toxicity.

### Example 15

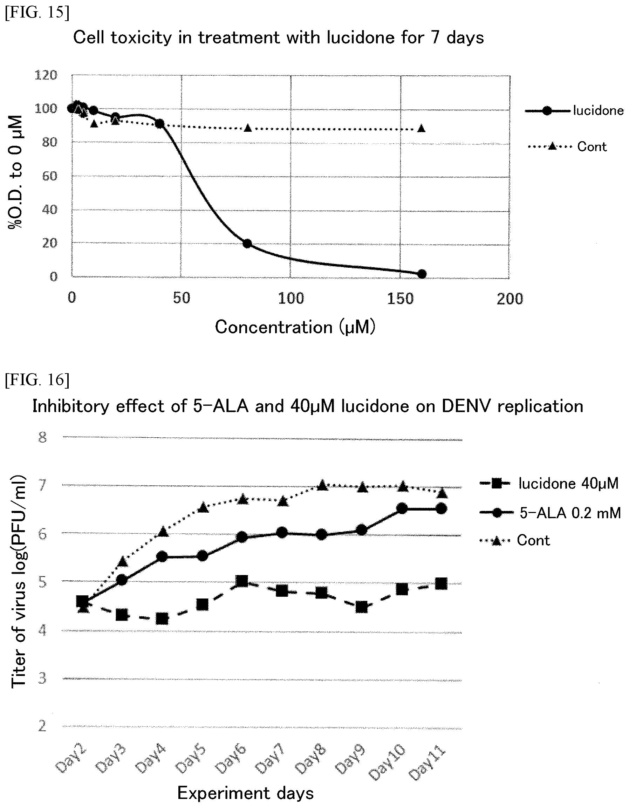

The cell toxicity caused by treatment with lucidone for 7 days was examined according to the above MTT assay. Fig. 15 shows the result of the cell toxicity caused to the non-infected cells treated with lucidone for 7 days. In Fig. 15, "Cont" represents a negative control. The cell toxicity of lucidone was observed in a dose-dependent manner. CC50 (a concentration of an agent at which cell toxicity is caused to 50% of cells) in treatment with lucidone for 7 days was higher than 60 µM. Lucidone did not cause the cell toxicity to the cells not infected with the flavivirus in a concentration of 40 µM. Thus, in the following examples, the concentration of lucidone for examining the anti-flavivirus activity was set to 40 µM.

### Example 16

In treatment with the agent for 7 days after infection, the inhibitory effect of 5-ALA on the dengue virus replication was compared with that of 40 µM lucidone. The agents in use were 5-ALA (0.2 mM), lucidone (40 µM), and water (Cont in figures). Treatment with the agent was performed from one day after infection for 7 days (from Day 2 to the end of Day 8). The titer of progeny viruses released from cells treated was evaluated and plotted on log(PFU/ml) as shown in Fig. 16. As shown in Fig. 16, 40 µM lucidone exhibited the stronger inhibitory effect on the dengue virus replication than 0.2 mM 5-ALA.

### Example 17

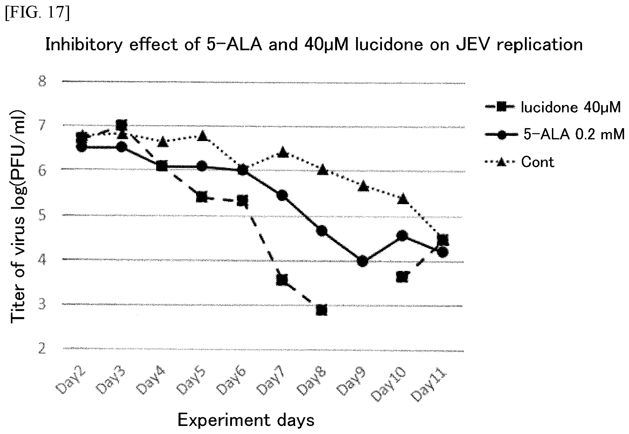

In treatment with the agent for 7 days after infection, the inhibitory effect of 5-ALA on the Japanese encephalitis virus replication was compared with that of 40 µM lucidone. The agents in use were 5-ALA (0.2 mM), lucidone (40 µM), and water (Cont in figures). Treatment with the agent was performed from one day after infection for 7 days (from Day 2 to the end of Day 8). The titer of progeny viruses released from cells treated was evaluated and plotted on log(PFU/ml) as shown in Fig. 17. As shown in Fig. 17, 40 µM lucidone exhibited the stronger inhibitory effect on the Japanese encephalitis virus replication than 0.2 mM 5-ALA.

### Example 18

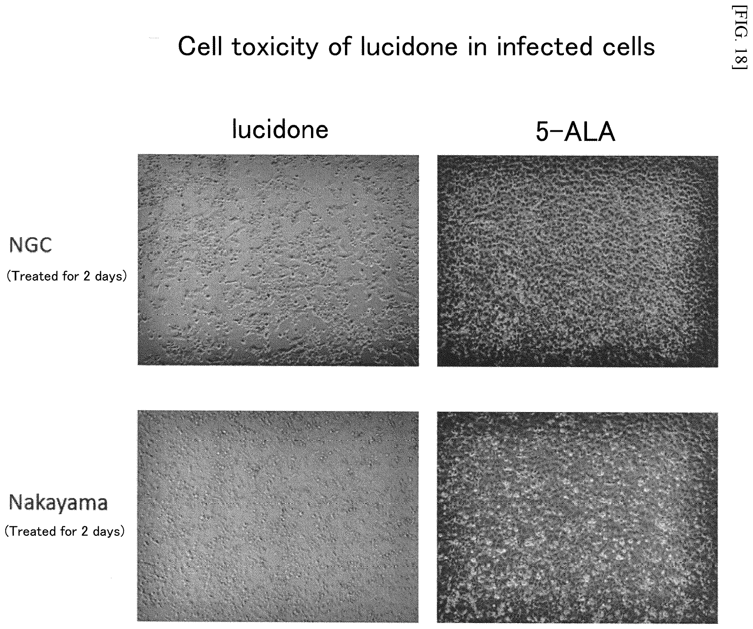

The cell toxicity of lucidone and 5-ALA caused to the flavivirus infected cells was examined. In the tests in Example 16 and Example 17, cells infected with the dengue virus type-2 New Guinea C strain (DENV2 NGC) or the Japanese encephalitis virus Nakayama strain (JEV Nakayama) were treated with the agent for 2 days, and the state of the cells was observed using an optical microscope. Fig. 18 shows a microscope photograph of each cell state. In Fig. 18, NGC represents the dengue virus type-2 New Guinea C strain (DENV2 NGC) and Nakayama represents the Japanese encephalitis virus Nakayama strain (JEV Nakayama). Both the dengue virus infected cells and the Japanese encephalitis virus infected cells treated with lucidone showed detachment in a significant population of the cells. This indicates that 40 µM lucidone exhibited the cell toxicity in the dengue virus infected cells and the Japanese encephalitis virus infected cells. On the other hand, the dengue virus infected cells and the Japanese encephalitis virus infected cells did not show detachment with 0.2 mM 5-ALA. That is, it is thought that 0.2 mM 5-ALA did not cause the cell toxicity to these infected cells.

Based on the foregoing experiments, it is speculated that the anti-flavivirus activity of 40 µM lucidone observed in Example 16 (i.e., Fig. 16) and Example 17 (i.e., Fig. 17) reflects the cell toxicity of lucidone in the infected cells. That is, lucidone kills the infected cells themselves serving as a host for the dengue virus and the Japanese encephalitis virus, thereby causing a reduction in the number of the host cells for virus proliferation in this experimental system. As a result, it is thought that the amount of the viruses present in the culture solution was reduced in the presence of lucidone. That is, it can be concluded that the results in Example 16 and Example 17 are not a result in which the anti-flavivirus activity of lucidone other than the cell toxicity is correctly compared with the anti-flavivirus activity of 5-ALA. The comparison of the anti-flavivirus activity between lucidone and 5-ALA should be performed in a concentration at which none of the agents cause the cell toxicity. Thus, in the following Example 19 and Example 20, the anti-flavivirus activity was compared between lucidone and 5-ALA in a concentration at which the cell toxicity is not caused.

### Example 19

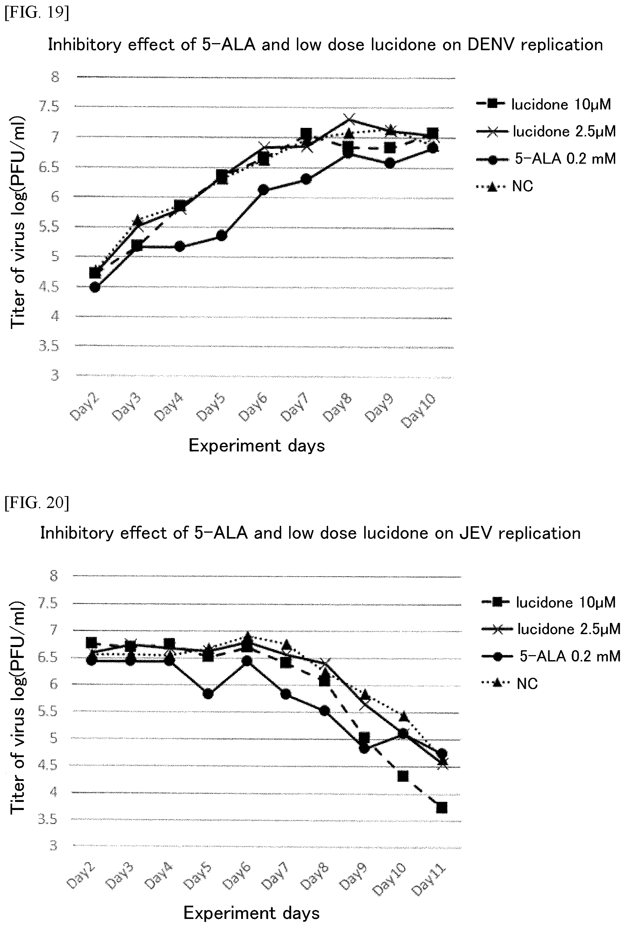

In treatment with the agent for 7 days after infection, the inhibitory effect of 5-ALA on the dengue virus replication was compared with that of 10 µM and 2.5 µM lucidone. The agents in use were 5-ALA (0.2 mM), lucidone (10 µM), lucidone (2.5 µM), and water (NC in figures). The treatment with the agent was performed from one day after infection for 7 days (from Day 2 to the end of Day 8). The titer of progeny viruses released from cells treated was evaluated and plotted on log(PFU/ml) as shown in Fig. 19. As shown in Fig. 19, 0.2 mM 5-ALA exhibited the stronger inhibitory effect on the dengue virus replication than 10 µM and 2.5 µM of lucidone.

### Example 20

In treatment with the agent for 7 days after infection, the inhibitory effect of 5-ALA on the Japanese encephalitis virus replication was compared with that of 10 µM and 2.5 µM lucidone. The agents in use were 5-ALA (0.2 mM), lucidone (10 µM), lucidone (2.5 µM), and water (NC in figures). Treatment with the agent was performed from one day after infection for 7 days (from Day 2 to the end of Day 8). The titer of progeny viruses released from cells treated was evaluated and plotted on log(PFU/ml) as shown in Fig. 20. As shown in Fig. 20, 0.2 mM 5-ALA exhibited the stronger inhibitory effect on the Japanese encephalitis virus replication than 10 µM and 2.5 µM of lucidone.

From the results in the Examples comparing lucidone and 5-ALA, a difference in concentrations for exhibiting cell toxicity and drug efficacy (i.e., a therapeutic window) is as follows.

The anti-flavivirus activity of 5-ALA is confirmed at 0.2 mM or higher, while causing the cell toxicity requires at least 0.8 mM (the cell toxicity of 5-ALA at higher than 0.8 mM is not examined). Thus, the therapeutic window of 5-ALA is 0.2 mM ≤ 5-ALA < 0.8 mM or higher. On the other hand, the anti-flavivirus activity of lucidone is confirmed at 40 µM, and the cell toxicity of lucidone is also confirmed at 40 µM, resulting in a lack of the therapeutic window. Thus, it is clear that 5-ALA is a more useful agent for preventing and/or treating the flavivirus infectious disease than lucidone. As one of action mechanisms of the anti-dengue virus activity of lucidone, an increase in HO-1 in cells is known. Thus, even if an increase in HO-1 in cells is known as anti-virus action of 5-ALA, it is obvious that the usefulness of 5-ALA as the agent for preventing and/or treating the flavivirus infectious disease is not solely caused by a putative increase in HO-1. It can be said that this advantageous effect of 5-ALA is an unexpected effect which is far beyond the effect solely caused by an increase in HO-1 as shown with lucidone.

### Example 21

In the same manner as in Example 6, 4 kinds of viruses, a dengue virus type-1 Mochizuki strain (DENV1), a dengue virus type-3 CH53489 strain (DENV3), a dengue virus type-4 TVP360 strain (DENV4), and a Zika virus PRVABC59 strain (ZKV) were used to confirm the inhibitory effect on the replication of each virus by treatment with the agent for 7 days after infection. It is noted that, on the day of infection (Day 1), inoculation of DENV1, DENV3, DENV4, and ZKV was performed at MOI = 0.2, MOI = 0.01, MOI = 0.01, and MOI = 0.05, respectively. Further, in the above procedure (3) of measuring the infection titer of the virus, the plates of the DENV1, DENV3, and DENV4 were left standing still at 37°C and 5% CO₂ for 10 days, while the plate of ZKV was left standing still for 6 days. It is noted that the agents in use were 5-ALA (0.2 mM), 5-ALA (0.04 mM), ribavirin (300 µM, Rib in figures), and water (NC in figures).

### (Example 21-1)

### Examination of inhibitory effect on dengue virus type-1 replication by treatment with agent for 7 days after infection

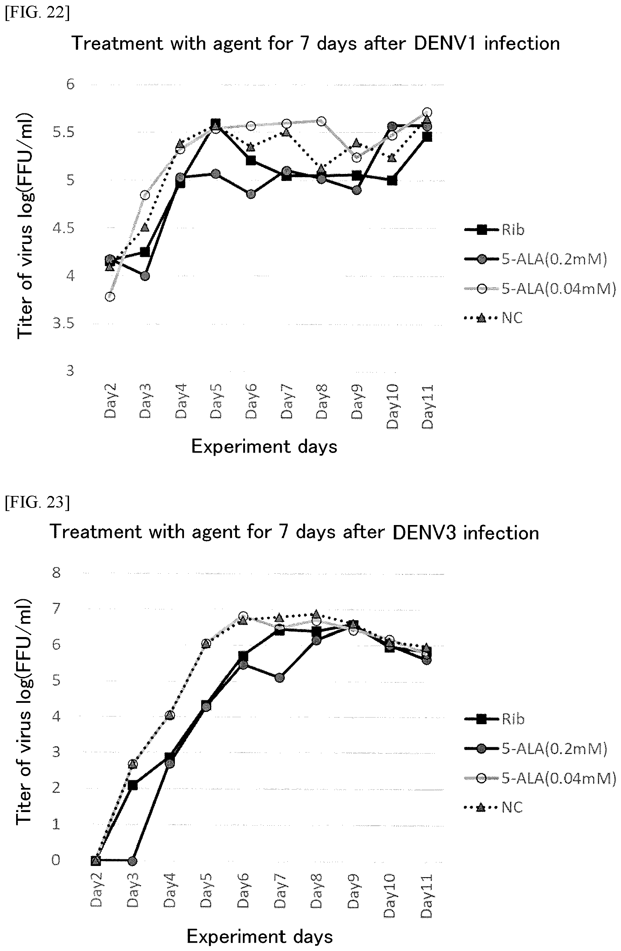

As shown in Fig. 22, the inhibitory effect on the dengue virus type-1 was exhibited up to Day 9 by treatment with 5-ALA (0.2 mM). The inhibitory effect on the dengue virus replication by treatment with 5-ALA (0.2 mM) was stronger than the effect of ribavirin (300 µM). When the agent was removed from the culture solution, this inhibitory effect was lost with the lapse of time.

### (Example 21-2)

### Examination of inhibitory effect on dengue virus type-3 replication by treatment with agent for 7 days after infection

As shown in Fig. 23, the inhibitory effect on the dengue virus type-3 was exhibited up to Day 7 by treatment with 5-ALA (0.2 mM). The inhibitory effect on the dengue virus replication by treatment with 5-ALA (0.2 mM) was stronger than the effect of ribavirin (300 µM). On Day 8, the inhibitory effect on the dengue virus replication by treatment with 5-ALA (0.2 mM) decreased. It is understood that the appearance of a 5-ALA resistance virus is one of the causes for the reduction in the inhibitory effect. The reduction in the inhibitory effect caused by continuous treatment with the agent was also observed in treatment with ribavirin from Day 7.

### (Example 21-3)

### Examination of inhibitory effect on dengue virus type-4 replication by treatment with agent for 7 days after infection

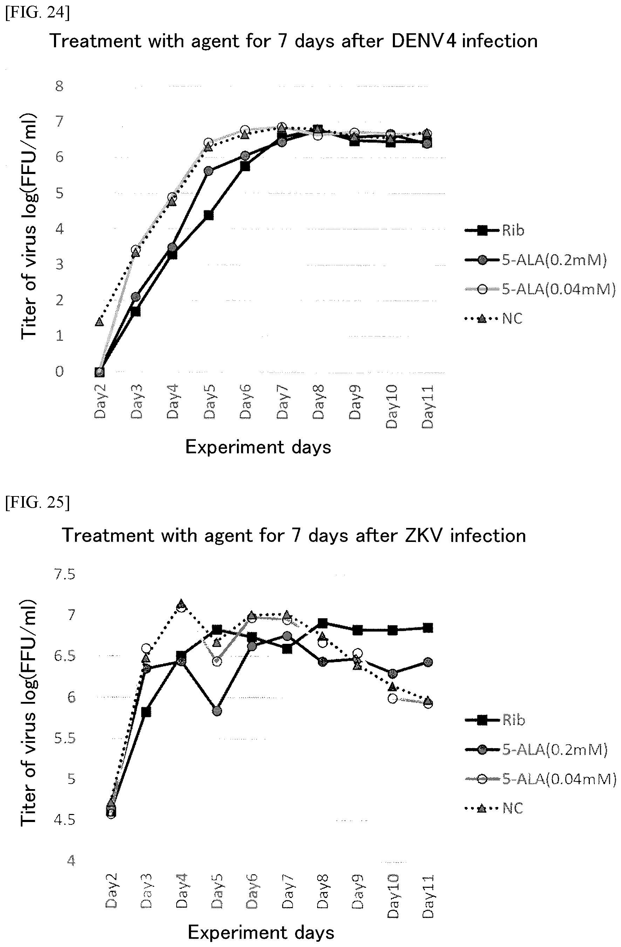

As shown in Fig. 24, the inhibitory effect on the dengue virus type-4 was exhibited up to Day 7 by treatment with 5-ALA (0.2 mM). The effect of ribavirin (300 µM) was stronger than the inhibitory effect on the dengue virus replication by treatment with 5-ALA (0.2 mM). On Day 8, the inhibitory effect on the dengue virus replication by treatment with 5-ALA (0.2 mM) decreased. It is thought that the appearance of a 5-ALA resistance virus is one of the causes for the reduction in the inhibitory effect. The reduction in the inhibitory effect caused by continuous treatment with the agent was also observed in treatment with ribavirin.

### (Example 21-4)

### Examination of inhibitory effect on Zika virus replication by treatment with agent for 7 days after infection

As shown in Fig. 25, the inhibitory effect on the Zika virus was exhibited up to Day 8 by treatment with 5-ALA (0.2 mM). The inhibitory effect on the Zika virus replication by treatment with 5-ALA (0.2 mM) was stronger than the effect of ribavirin (300 µM) on Day 5, Day 6, and Day 8. After Day 9, the inhibitory effect on the dengue virus replication by treatment with 5-ALA (0.2 mM) decreased. It is thought that the appearance of a 5-ALA resistance virus is one of the causes for the reduction in the inhibitory effect. The reduction in the inhibitory effect caused by continuous treatment with the agent was also observed in treatment with ribavirin.

### Industrial Applicability

The agent for preventing and/or treating the flavivirus infectious disease of the embodiment of the present invention, comprising at least one selected from 5-aminolevulinic acid (5-ALA) or the ester thereof, or the salt thereof, and the method for preventing and/or treating the flavivirus infectious disease of the embodiment of the present invention can be used to prevent and/or treat the flavivirus infectious disease.

## Claims

1. An agent for preventing and/or treating a flavivirus infectious disease comprising at least one selected from 5-aminolevulinic acid (5-ALA) or an ester thereof, or a salt thereof.

2. The agent for preventing and/or treating the flavivirus infectious disease according to claim 1, wherein a flavivirus is a dengue virus, a Zika virus, a Japanese encephalitis virus, a tick-borne encephalitis virus, a West Nile virus, and a yellow fever virus.

3. The agent for preventing and/or treating the flavivirus infectious disease according to claim 1, further comprising an iron compound.

4. The agent for preventing and/or treating the flavivirus infectious disease according to claim 1, wherein light irradiation is not required.

5. A method for preventing and/or treating a flavivirus infectious disease, comprising administering an agent for preventing and/or treating the flavivirus infectious disease comprising at least one selected from 5-aminolevulinic acid (5-ALA) or an ester thereof, or a salt thereof to a subject.

6. The method for preventing and/or treating the flavivirus infectious disease according to claim 5, wherein a flavivirus is a dengue virus, a Zika virus, a Japanese encephalitis virus, a tick-borne encephalitis virus, a West Nile virus, and a yellow fever virus.

7. The method for preventing and/or treating the flavivirus infectious disease according to claim 5, wherein the agent for preventing and/or treating the flavivirus infectious disease further comprises an iron compound.

8. The method for preventing and/or treating the flavivirus infectious disease according to claim 5, wherein light irradiation is not required.
